# EUROPÄISCHE PATENTSCHRIFT

(11) **EP 1 341 778 B1**
(45) Veröffentlichungstag und Bekanntmachung des Hinweises auf die Patenterteilung: **24.05.2006**
(21) Anmeldenummer: 01989528.3
(22) Anmeldetag: 28.11.2001
(51) Int. Cl.: C07D 311/32, C07C 49/835, C07C 49/84, A61K 31/353, A61K 31/12, A61P 25/00

(54) **KLAINETINE UND IHRE DERIVATE, VERFAHREN ZU IHRER HERSTELLUNG UND VERWENDUNG DERSELBEN**
KLAINETIN AND DERIVATIVES THEREOF, METHOD FOR THEIR PRODUCTION AND THE USE OF THE SAME
KLAINETINES ET LEURS DERIVES, PROCEDE DE PRODUCTION ET UTILISATION DE CES COMPOSES

(30) Priorität: 05.12.2000 DE 10060677
(43) Veröffentlichungstag der Anmeldung: 10.09.2003
(73) Patentinhaber: Sanofi-Aventis Deutschland GmbH, 65929 Frankfurt am Main (DE)
(72) Erfinder: HAAG, Sabine, 60322 Frankfurt (DE); KOGLER, Herbert, 61479 Glashütten (DE); LI, Ziyu, 63067 Offenbach (DE); VERTESY, Laszlo, 65817 Eppstein-Vockenhausen (DE)
(86) Internationale Anmeldenummer: PCT/EP2001/013873
(87) Internationale Veröffentlichungsnummer: WO 2002/046180

(56) Entgegenhaltungen:
- WO-A-98/58913
- LASSWELL W.L. JR. ET AL.: "Cytotoxic C-benzylated flavonoids from Uvaria chamae" JOURNAL OF ORGANIC CHEMISTRY., Bd. 42, Nr. 8, 1977, Seiten 1295-1302, XP002197367 AMERICAN CHEMICAL SOCIETY. EASTON., US ISSN: 0022-3263
- HUFFORD C.D. ET AL.: "Uvaretin and Isouvaretin. Two novel cytotoxic C-benzylflavanones from Uvaria chamae L." JOURNAL OF ORGANIC CHEMISTRY., Bd. 41, Nr. 7, 1976, Seiten 1297-1298, XP002197368 AMERICAN CHEMICAL SOCIETY. EASTON., US ISSN: 0022-3263
- COLE J.R. ET AL.: "Uvaretin, a new antitumor agent from Uvaria acuminata (Annonaceae)" JOURNAL OF ORGANIC CHEMISTRY., Bd. 41, Nr. 10, 1976, Seiten 1852-1855, XP002197369 AMERICAN CHEMICAL SOCIETY. EASTON., US ISSN: 0022-3263
- HUFFORD C.D. ET AL.: "Uvarinol: a novel cytotoxic tribenzylated flavanone from Uvaria chamae" JOURNAL OF ORGANIC CHEMISTRY., Bd. 44, Nr. 25, 1979, Seiten 4709-4710, XP002197370 AMERICAN CHEMICAL SOCIETY. EASTON., US ISSN: 0022-3263

## Beschreibung

Die vorliegende Erfindung betrifft neue Verbindungen der allgemeinen Formel I 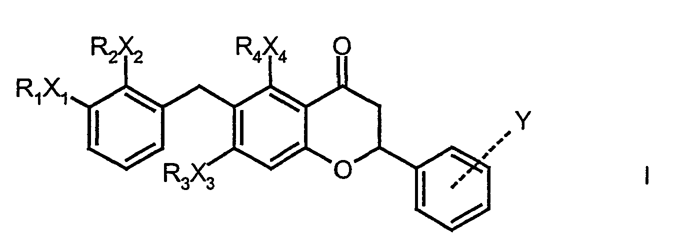 und Formel II 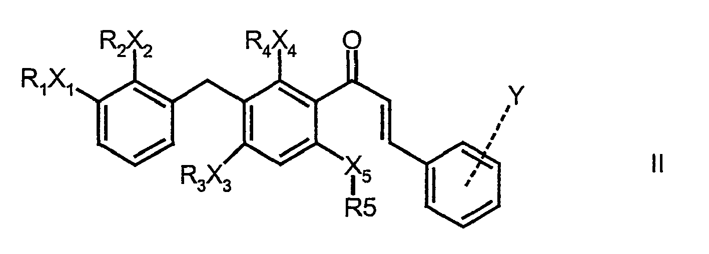 worin R₁, R₂, R₃, R₄,R₅, X₁, X₂, X₃, X₄, X₅ sowie Y die Bedeutung wie unten beschrieben haben. Die Verbindungen der Formel I und II hemmen Cyclin-abhängige Kinasen [cyclin-dependent kinases (CDKs)] und weitere Kinasen (z.B. KDR), besitzen cytostatische Wirkungen und sind besonders geeignet zur Behandlung von Tumoren. Die Verbindungen der Formel I und II sind erhältlich durch Extraktion der Pflanze Uvaria klaineri, PLA 100484, durch Zellkulturen der Pflanze Uvaria klaineri oder durch chemische Synthese. Die Erfindung betrifft somit ein Verfahren zur Herstellung der Verbindungen der Formel I und II, die Verwendung der Verbindungen zur Herstellung eines Arzneimittels zur Behandlung von malignen Erkrankungen und von Krankheiten die durch Hemmen von CDKs und KDR behandelt werden können, sowie pharmazeutischen Zubereitungen mit einem Gehalt an mindestens einer Verbindung der Formel I oder II.

Krebs ist eine zumeist tödlich verlaufende Erkrankung von Mensch und Tier, die durch das unkontrollierte Wachsen von körpereigenen Zellen verursacht wird. Krebs ist die Bezeichnung für die Bildung von bösartigen Geschwülsten (Malignome), von Neoplasmen (Tumoren bzw. Carcinoma) oder für die maligne Entartung sowie Reifungsstörung weißer Blutzellen (Leukämie, Blutkrebs). Krebs- oder Tumorzellen entstehen durch Umwandlung körpereigener Zellen. Die Bösartigkeit der Krebszelle drückt sich in der Autonomie des Wachstums aus, das heißt in ihrer Fähigkeit, ungehemmt und ohne Einordnung in den Bauplan der Organe und unter Gewebszerstörung infiltrierend zu wachsen. Ein sicheres Zeichen der Malignität ist die Bildung tumorfemer Absiedlungen (Metastasen) nach hämatogener oder lymphogener Ausbreitung von Tumorzellen. Krebs gehört zu den häufigsten Todesursachen des Menschen und deshalb besteht ein großer Bedarf an Methoden und Mitteln zur Heilung oder Behandlung von malignen Entartungen.

Die Möglichkeit einer Therapie maligner Tumoren umfasst neben der - wenn möglich radikalen - operativen Entfernung des Tumors, die radiologische Therapie mit Röntgenstrahlen, α-, β-, γ-Strahlen, die Immuntherapie und die Chemotherapie. Die Immuntherapie ist derzeit nur in eingeschränktem Maß anwendbar. Unter der Chemotherapie von Tumoren versteht man die Gabe von Zellgiften (Cytostatika) zur Behandlung von Tumoren und von verbliebenen Tumorzellen nach lokaler chirurgischer Behandlung oder Bestrahlung. Diese Stoffe greifen spezifisch in bestimmte Vorgänge der Zellteilung ein, so dass Gewebe mit hohem Anteil an sich teilenden Zellen, wie das rasch wachsende Tumorgewebe, empfindlicher reagieren. Zum Einsatz kommen alkylierende Verbindungen, wie z. B. das Cyclophosphamid (The Merck Index, 12. Ed. Seite 463), Antimetabolite, wie das Methotrexat (The Merck Index, 12. Ed. Seite 1025), Alkaloide, wie das Vincristin (The Merck Index, 12. Ed. Seite 1704) und Antibiotika, wie das Daunomycin (The Merck Index, 12. Ed. Seite 479) sowie das Adriamycin (The Merck Index, 12. Ed. Seite 581-582). All diese Agenzien haben aber aufgrund von massiven Nebenwirkungen große Nachteile, so dass der Tod der Erkrankten nur hinausgezögert, nicht jedoch abgewendet wird. Zudem treten bei entarteten (Krebs-) Zellen Resistenzen gegen die angewandten Mittel auf, die derzeitigen Medikamente wirken dann nicht mehr cytostatisch, wohl aber toxisch infolge der Nebenwirkungen. Zudem hat sich gezeigt, dass eine kombinierte bzw. sequentielle Anwendung von Cytostatika die Wirksamkeit eines einzelnen Cytostatikums (Monotherapie) übertrifft und dadurch möglich ist, dass sich die erheblichen Nebeneffekte bei der Polychemotherapie nicht addieren. Aus all diesen Gründen werden neue Chemotherapeutika dringend benötigt und deshalb weltweit gesucht.

Es ist überraschend gefunden worden, dass die afrikanische Pflanze Uvaria klaineri hoch wirksame neue Cytostatika zu bilden vermag, die das Wachstum von Zellen noch in sehr geringen Konzentrationen hemmen. Die neuen Verbindungen werden im folgendem Klainetine genannt und sind, zusammen mit Klainetin-Derivaten, Gegenstand der Erfindung. Die Klainetine sind substituierte Phenole, die die cyclin-abhängigen Kinasen hemmen und dadurch die Regulation des Zell-Zyclus beeinflussen. Da bei Krebserkrankungen die Zell-Vermehrung krankhaft beschleunigt und die Regulation außer Kontrolle geraten ist, sind CDK-Inhibitoren wertvolle Agenzien zur Behandlung maligner Entartungen. Die Verbindungen der Formel I gehören zum Typ der substituierten 2,3-Dihydro-flavone, das Ringgerüst entspricht dem des Isochamanetins, welches zuletzt von H. Achenbach et al. in Phytochemistry, Band 44, Seite 359-364, 1997 beschrieben worden ist. Die erfindungsgemäßen Verbindungen der Formel I sind neuartige, substituierte Isochamanetine.

Die Verbindungen der Formel II gehören zum Verbindungsklasse der Chalkone, das Ring- und Kohlenstoff-Gerüst entspricht dem des Uvaretins. Das Uvaretin ist in vielen Uvaria-Arten gefunden worden (M. H. H. Nkunya et al. Planta Med. 57, 341-343,1991; I. Muhammad & P. G. Waterman, J. Nat. Prod. 48, 571-80, 1985). Die erfindungsgemäßen Verbindungen der Formel II unterscheiden sich durch Substitution und Dehydrierung von dem Uvaretin, sie sind in der Literatur noch nicht beschrieben worden. Aufgrund ihre abweichende chemische Struktur besitzen die Verbindungen der Formeln I und II neue und physiko-chemische, biologische und pharmakologische Eigenschaften. Die bisher bekannten Substanzen weisen demgegenüber oft Nachteile auf, die sich in unbefriedigender Wirkhöhe, hoher Toxizität und/oder unerwünschten Nebenwirkungen äußern.

Die vorliegende Erfindung betrifft daher Verbindungen der allgemeinen Formel I 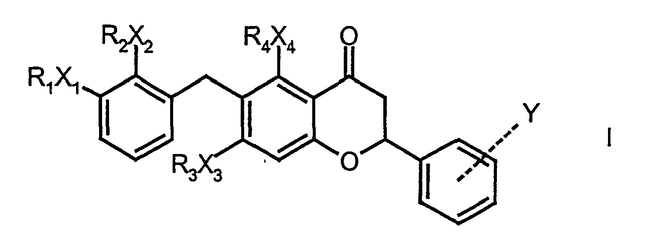 und der Formel II 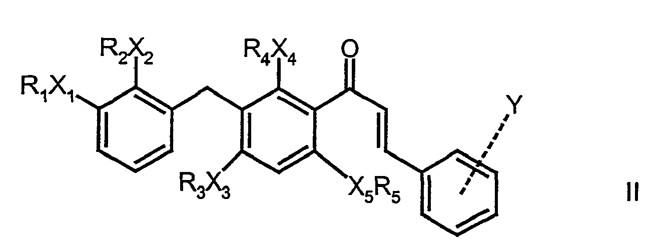 worin
R₁, R₂, R₃, R₄ und R₅ unabhängig von einander, jeweils H, C₁-C₆-alkyl, C₂-C₆-alkenyl oder C₂-C₆-Alkinyl, gegebenenfalls mit einem oder mehreren OH substituiert;
X₁, X₂ ,X₃, X₄ und X₅ unabhängig voneinander O, NH, N-C₁-C₆-Alkyl, N-C₂-C₆-Alkenyl, N-C₂-C₆-Alkinyl, Acyl, Aryl oder S;
Y eine oder mehrere H, Halogen, OH, O-C₁-C₆-Alkyl, O-C₂-C₆-Alkenyl, O-C₂-C₆₋Alkinyl, NH₂, NH-C₁-C₆-Alkyl, NH-C₂-C₆-Alkenyl, NH-C₂-C₆-Alkinyl, NH-acyl, SH, S-C₁₋C₆-Alkyl, S-C₂-C₆-Alkenyl, S-C₂-C₆-Alkinyl, Acyl oder Aryl; bedeuten,
in allen ihren stereochemischen Formen und Gemische dieser Formen in jedem Verhältnis, sowie deren physiologisch verträgliche Salze.

In den Formeln I und II bedeuten
C₁-C₆-Alkyl ein geradkettiges oder verzweigtes Alkyl mit 1 bis 6 C-Atomen, wie z.B. Methyl, Ethyl, i-Propyl, tert.Butyl und Hexyl,
C₂-C₆-Alkenyl ein geradkettiges oder verzweigtes Alkenyl mit 2 bis 6 C-Atomen, wie z.B. Allyl, Crotyl und Pentenyl, und
C₂-C₆-Alkinyl ein geradkettiges oder verzweigtes Alkinyl mit 2 bis 6 C-Atomen, wie z.B. Propinyl, Butinyl und Pentinyl.

Aryl kann z.B. für Phenyl, Benzyl oder 1- oder 2- Naphthyl, daß auch noch substituiert sein kann, beispielsweise durch Halogen, wie Chlor, Brom, Fluor, durch Alkyl mit 1-4 C-Atomen, vorzugsweise Methyl-, durch Hydroxy, durch Alkoxy mit 1-4 C Atomen, insbesondere Methoxy und/oder durch Trifluormethoxy stehen.

Acyl kann für aliphatische oder aromatische Acyl-Reste stehen. Aliphatisches Acyl hat 1-7, vorzugsweise 1-4 C Atome, wie z.B. Formyl, Acetyl, Propionyl, Butyryl, Hexanoyl, Acryloyl, Crotonoyl, Propioloyl, das noch weiter substituiert sein kann, beispielsweise durch Halogen, wie Chlor, Brom, Fluor, durch Amino, und/oder durch Alkylamino mit 1-4 C Atomen, vorzugsweise Methyl- oder Ethylamino-Gruppen. Aromatisches Acyl kann z.B. Benzoyl oder Naphthoyl sein, das auch noch weiter substituiert sein kann, beispielsweise durch Halogen, wie Chlor, Brom, Fluor, durch Alkyl mit 1-4 C-Atomen, vorzugsweise Methyl, durch Hydroxy-, durch Amino-Gruppen, wie z.B. Ethylamino, oder durch Alkoxy-Gruppen mit 1-7, vorzugsweise 1-4 C Atomen, insbesondere Methoxy.

In der Formel I bedeuten X₁R₁, X₂R₂ und X₃R₃ vorzugsweise OH, X₄R₄ vorzugsweise O-C₁-C₄-Alkyl und Y H; in der Formel II bedeuten X₁R₁, X₂R₂, X₃R₃, sowie X₄R₄ vorzugsweise OH, X₅R₅ vorzugsweise O-C₁-C₄-Alkyl und Y H.

Die Erfindung betrifft somit das Klainetin A der Formel IA

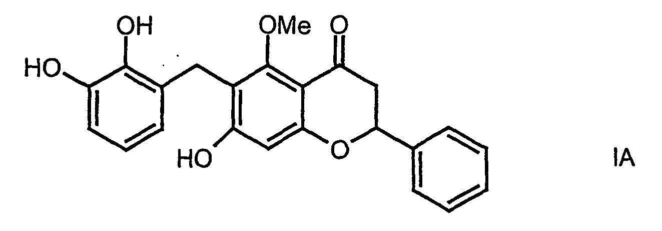

in allen ihren stereochemischen Formen und Gemische dieser Formen in jedem Verhältnis, sowie deren physiologisch verträgliche Salze.

Die Erfindung betrifft somit weiter das Klainetin B der Formel IIA

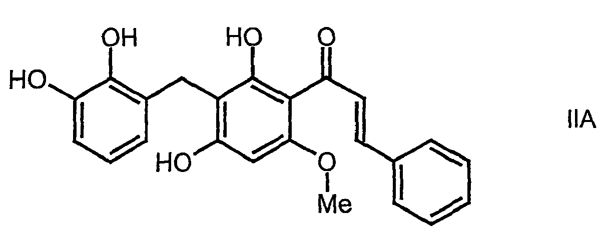

Erfindungsgemäß sind die Verbindungen der Formel I erhältlich durch Extraktion der Pflanze Uvaria klaineri oder eines ihrer Varianten oder Mutanten unter geeigneten Bedingungen. Durch anschließende Isolierung der Verbindungen und gegebenenfalls Umwandlung in chemische Derivate, sowie deren physiologisch verträglichen Salze werden die Klainetine gewonnen.

Die Erfindung betrifft daher weiterhin ein Verfahren zur Herstellung einer Verbindung der Formel I und/oder II, dadurch gekennzeichnet, dass die Pflanze Uvaria klaineri oder einer ihrer Varianten oder Mutanten unter geeigneten Bedingungen gezüchtet wird, bis sich ein oder mehrere Klainetine der Formel IA und/oder IIA in dem Pflanzenmatrial anhäufen und anschließend aus den Pflanzenteilen isoliert und gegebenenfalls in chemische Derivate und /oder physiologisch verträglichen Salze umgewandelt werden.

Vorzugsweise wird die Pflanze Uvaria klaineri, ihre Mutanten und/oder Varianten auf geeigneten Böden im tropischem oder subtropischem Klima gezüchtet, bis sich die neuen Klainetine in der Pflanze anhäufen, anschließend werden die Klainetine aus den Pflanzen isoliert und gegebenenfalls in die einzelnen aktiven Komponenten aufgetrennt.

Die Pflanzenproduktion wird vorzugsweise unter tropischen Bedingungen durchgeführt, sie verläuft besonders gut bei einer Temperatur zwischen 18 und 45 C und bei hoher Luftfeuchtigkeit.

Eine andere Möglichkeit ist die Produktgewinnung durch Zellkulturen. Hierzu werden lebende Zellen der Pflanze Uvaria klaineri in eine geeignete Nährlösung übertragen und die Zellkulturen gezüchtet, bis sich in dem Medium die erfindungsgemäßen Verbindungen anhäufen. Zellkulturen werden vorzugsweise aus Kalluskulturen angelegt. Die Nährmedien enthalten Mineralien und Vitaminen, sowie KohlenstoffQuellen, z. B. Saccharose und Stickstoff Quellen, wie z. B. Stickstoff-Salzen. Anschließend werden die Klainetine aus den Zellkultur isoliert und gegebenenfalls in die einzelnen aktiven Komponenten aufgetrennt.

In der Literatur sind viele Reaktionen zur chemischen Abwandlung von Flavonen oder Phenolen beschrieben worden. Die Derivatisierung der phenolischen OH-Gruppen der vorliegenden Verbindungen kann daher mit an sich bekannten chemischen Reaktionen durchgeführt werden. Eine Reduktion zu den gesättigten Verbindungen kann zum Beispiel mit Wasserstoff katalytisch erreicht werden oder die Reduktion von Carbonyl-Gruppen kann mit Metall-Hydriden, wie Aluminium-Hydriden oder Bor-Hydriden erfolgen. Als ein weiteres Beispiel kommt die Umsetzung von Carbonyl-Gruppen mit Hydroxylamin oder mit seinen Derivaten zu den Oximen in Betracht, die ihrerseits weiter chemisch umgewandelt werden können.

Im folgenden wird die Erfindung detailliert beschrieben, insbesondere in ihren bevorzugten Ausführungsformen.
Die erfindungsgemäßen Klainetine werden durch Uvaria klaineri produziert.

Uvaria klaineri ist eine weit verbreitete immergrüne Liane aus der Familie der Annonaceae. In der Familie der Annonaceae finden sich viele tropische Nutz- und Heilpflanzen. Das Verbreitungsgebiet sind die gesamten Tropen, die Probe aus der die Klainetine isoliert worden sind, wurde in Gabun, speziell in der Gegend um Rabi/Gamba gesammelt.

Zur Isolierung der Klainetine können auch andere Arten aus der Gattung Uvaria verwendet werden, bzw. auch Pflanzen derselben Spezies, die von einem anderen Standort stammen. Gehalt und Zusammensetzung an Klainetinen können je nach Standortverhältnissen, wie z.B. Bodenbeschaffenheit, Temperatur, Feuchtigkeit, Lichteinfall variieren.

Das erfindungsgemäße Verfahren kann für die Extraktion und Isolierung im Labormaßstab 100 g bis 1 kg (trockenes Pflanzenmaterial) und industriellen Maßstab (100 bis > 1000 kg) eingesetzt werden.

In dem Pflanzenmaterial bildet Uvaria klaineri ein Gemisch aus Klainetinen. Je nach Ernte der Pflanze und ihrer Teile kann der mengenmäßige Anteil eines oder mehrerer der erfindungsgemäßen Klainetine variieren. Außerdem kann durch die Züchtungsbedingungen die Synthese einzelner Klainetine gesteuert werden, so dass eines oder auch mehrere der Klainetine gar nicht bzw. in einer Menge unterhalb der Nachweisgrenze von der Pflanze gebildet werden.

Vorzugsweise enthält das Pflanzenmaterial ein nachweisbares Klainetin. Bevorzugt werden die Klainetine A oder B gebildet.

Neben den Klainetinen A und B (Verbindungen der Formel IA bzw. IIA) werden in der Pflanze Uvaria klaineri auch weiter verwandte Verbindungen gebildet, die sich durch eine veränderte Hydroxylierung oder Glycosylierung von den in den Formeln IA und IIA wiedergegebenen Verbindungen unterscheiden. So wurde als Nebenprodukt ein weiteres Klainetin mit dem Molekulargewicht 408 Da nachgewiesen.

Die Kultivierung der Pflanzen kann im Freiland oder vorzugsweise im Gewächshaus erfolgen, alternativ können pflanzliche Zellkulturen zur Produktion der Metabolite eingesetzt werden. Das Ausgangsmaterial besteht in der Regel aus Kalluskulturen. Durch die Auswahl geeigneter Bioreaktoren zur Züchtung der pflanzlichen Zellkultur kann eine optimale Durchmischung und Belüftung der Kultur ohne die Einwirkung von zu starken Scherkräften auf die Pflanzenzellen, das Zellwachstum und die Metaboliten-Produktion erzielt werden. Beispielsweise können Airlift- oder Blasensäulenreaktoren, sowie Blatt- oder Propellerrührwerke zur Durchmischung der Kulturen eingesetzt werden. Die Zellen können als Einzelzellen bzw. verzweigte oder unverzweigte Zell-aggregate oder -ketten wachsen. Die Metabolitproduktion kann durch Stimulation mit exogenen Faktoren, z.B. Schwermetallsalzen oder pflanzlichen Elicitoren induziert werden.

Die Produktbildung in der Pflanze kann anhand des pH-Wertes der Kulturen sowie durch chromatographische Methoden, wie z.B. Dünnschichtchromatographie oder High Pressure Liquid Chromatography oder Ausprüfen der biologischen Aktivität überwacht werden. Die erfindungsgemäßen Klainetine sind sowohl im Blattwerk als auch anderen Pflanzenteilen enthalten. Das im folgenden beschriebene Isolierungsverfahren dient zur Aufreinigung der erfindungsgemäßen Klainetine, vorzugsweise zur Aufreinigung der Klainetine A und B.

Die Isolierung bzw. Aufreinigung der erfindungsgemäßen Klainetine aus der Pflanze oder dem Kulturmedium erfolgt nach bekannten Methoden unter Berücksichtigung der chemischen, physikalischen und biologischen Eigenschaften der Naturstoffe. Zum Testen der Klainetin-Konzentrationen im Ausgangsmaterial oder in den einzelnen Isolierungsstufen kann die Dünnschichtchromatographie, beispielsweise an Kieselgel mit Chloroform-Methanol-Eisessig-Wasser- Gemischen (z. B. im Mengenverhältnis 8:1:1:0,2) als Laufmittel oder HPLC verwendet werden. Die Detektion bei der dünnschichtchromatographischen Auftrennung kann beispielsweise durch Färbereagentien wie J₂-Dampf, Eisen(III)-chlorid, Vanillin-H₂SO₄ oder Pauli's Reagenz (Sulfanilsäure, diazotiert) erfolgen, wobei die Menge der gebildeten Substanz zweckmäßig mit einer Eichlösung verglichen wird.

Zur Isolierung der erfindungsgemäßen Klainetine wird die Pflanze geerntet, d. h. zunächst die Blätter, Stengel, Holz, die Rinde oder Wurzeln gesammelt, noch in frischem Zustand oder getrocknet nach den üblichen Verfahren extrahiert, und anschließend werden die Klainetine aus dem Pflanzenmaterial mit einem gegebenenfalls wasserhaltigem organischen Lösungsmittel extrahiert. Die organische Lösungsmittelphase enthält die erfindungsgemäßen Klainetine, sie werden gegebenenfalls im Vakuum konzentriert und wie unten beschrieben weiter aufgereinigt.

Die Extrakte werden vereinigt, mit Wasser verdünnt und mit einem geeigneten, mit Wasser nicht mischbarem organischen Lösungsmittel, zum Beispiel mit n-Butanol, extrahiert. Die anschließend abgetrennte organische Phase wird ggf. im Vakuum konzentriert. Man kann das Konzentrat zum Entfetten des Wertproduktes mit einem unpolaren Lösungsmittel, in dem die erfindungsgemäßen Klainetine sehr wenig löslich sind, wie zum Beispiel mit Hexan, Petroleum-ether, Diethylether verdünnen. Hierbei präzipitieren die Kainetine, die lipophilen Verunreinigungen, wie Wachse, bleiben gelöst und werden durch übliche Fest/flüssig-Phasentrennungen entfernt. Der Niederschlag, der die gesamten Klainetine enthält, wird in 1/30 des ursprünglichen Volumens Wasser/Methanol gelöst. Der Niederschlag geht dabei in Lösung und wird lyophilisiert. Das Lyophilisat, in der Folge als Rohprodukt bezeichnet, enthält 0,5 bis 10 % Klainetine und wird für die weitere Isolierung eingesetzt.

Die weitere Aufreinigung eines oder mehrerer der erfindungsgemäßen Klainetine erfolgt durch Chromatographie an geeigneten Materialien, vorzugsweise z.B. an Molekularsieben, an Normal-Phasenträger, wie z. B. Kieselgel, Aluminiumoxid, an lonenaustauschem oder an Adsorberharzen bzw. an Umkehr-Phasen (reversed phase, RP). Mit Hilfe dieser Chromatographie werden die Klainetine getrennt. Die Chromatographie der Klainetine erfolgt mit gepufferten wässrigen Lösungen oder Gemischen von wässrigen und organischen Lösungen.

Unter Gemischen von wässrigen und organischen Lösungen versteht man alle mit Wasser mischbaren organischen Lösemittel, vorzugsweise Methanol, 2-Propanol und Acetonitril, in einer Konzentration von 10 bis 80 % Lösemittel, vorzugsweise 15 bis 55 % Lösemittel, oder auch alle gepufferten wässrigen Lösungen, die mit organischen Lösemitteln mischbar sind. Die zu verwendeten Puffer sind die gleichen wie oben angegeben.

Die Trennung der Klainetine aufgrund ihrer unterschiedlichen Polarität erfolgt mit Hilfe der Reversed Phase Chromatographie, beispielsweise an MCI® (Adsorberharz von Mitsubishi, Japan) oder Amberlite XAD® (TOSOHAAS), an weiteren hydrophoben Materialien, wie zum Beispiel an RP-8- oder RP-18-Phasen aber auch an sog. Polyamiden. Außerdem kann die Trennung mit Hilfe der Normalphasen-Chromatographie, zum Beispiel an Kieselgel, Aluminiumoxyd und ähnlichen erfolgen.

Die Chromatographie der Klainetine erfolgt mit gepufferten oder angesäuerten wässrigen Lösungen oder Gemischen von wässrigen Lösungen mit Alkoholen oder anderen, mit Wasser mischbaren organischen Lösemitteln. Als organisches Lösemittel wird vorzugsweise Propanol und Acetonitril verwendet.

Unter gepufferten oder angesäuerten wässrigen Lösungen versteht man z.B. Wasser, Phosphatpuffer, Ammoniumacetat, Citratpuffer in einer Konzentration von 1 mM bis 0,5 M sowie Ameisensäure, Essigsäure, Trifluoressigsäure oder allen handelsüblichen, dem Fachmann bekannten Säuren, vorzugsweise in einer Konzentration von 0,01 bis 3 %, insbesondere 0,1 %.

Chromatographiert wird mit einem Gradienten, der mit 100 % wässrigem Puffer beginnt und mit 100 % Lösemittel endet, vorzugsweise wird ein linearer Gradient von 10 bis 50 % 2-Propanol oder Acetonitril gefahren.

Altemativ kann auch eine Gelchromatographie oder die Chromatographie an hydrophoben Phasen erfolgen.

Die Gelchromatographie wird an Polyacrylamid- oder Mischpolymergelen durchgeführt, wie z.B. Biogel-P 2® (Fa. Biorad), Fractogel TSK HW 40® (Fa. Merck, Deutschland oder Toso Haas, USA) oder an Sephadex® (Fa. Pharmacia, Uppsala, Schweden).

Die Reihenfolge der vorgenannten Chromatographien ist umkehrbar.

Ein weiterer, sehr wirksamer Reinigungsschritt für Klainetine ist die Kristallisation. Die Klainetine kristallisieren aus Lösungen in organischen Lösungsmitteln und aus Mischungen von Wasser mit organischen Lösungsmitteln. Die Kristallisation wird in an sich bekannter Weise, zum Beispiel durch Konzentrieren oder Abkühlen gesättigter Klainetin-Lösungen, durchgeführt.

Die erfindungsgemäßen Klainetine sind im festen Zustand und in Lösungen im pH-Bereich zwischen 3 und 8, insbesondere 4 und 6, stabil und lassen sich damit in übliche galenische Zubereitungen einarbeiten.

Die Klainetine und abgeleitete chemische Derivate der Formel I und II können nach dem Fachmann bekannten Methoden in die entsprechenden physiologisch verträglichen Salzen übergeführt werden.

Unter physiologisch verträglichen Salzen von Verbindungen der Formel I und II versteht man sowohl deren organische als auch anorganische Salze, wie sie in Remington's Pharmaceutical Sciences (17. Auflage, Seite 1418 (1985)) beschrieben sind. Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Ester-, und Ether-Derivate sowie Reduktionsprodukte können nach in der Literatur beschriebenen Verfahren, z.B., in "Advanced Organic Synthesis, 4^{th} Edition, J. March, John Wiley & Sons., 1992, hergestellt werden.

Die vorliegende Erfindung umfasst allen stereoisomeren Formen der Verbindungen der Formel I und II. In den Verbindungen der Formel IA und IIA enthaltene Asymmetriezentren können alle unabhängig voneinander die S-Konfiguration oder die R-Konfiguration aufweisen. Zur Erfindung gehören alle möglichen Enantiomeren und Diastereomeren, ebenso Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als linksdrehende als auch als rechtsdrehende Antipoden, R- und S-Konfigurationen, in Form von Racematen und in Form von Mischungen der beiden Enantiomeren in allen Verhältnissen Gegenstand der Erfindung. Bei Vorliegen einer cis/trans-isomerie sind sowohl die cis-Form als auch die trans-Form und Gemische dieser Formen in allen Verhältnissen Gegenstand der Erfindung.

Tests zur Ermittlung der biologischen Aktivitäten der Klainetine:
Bei den Testsystemen handelt es sich um eine Kinase der CDK-Familie, CDK4 (Cyclin-abhängige Kinasen) sowie um KDR (Tyrosin-Kinase-Rezeptor), die eine wichtige Rolle in der Regulation des Zellzyklus und der Angiogenese spielen. CDK4 leitet durch Phosphorylierungsreaktionen eine Reaktionskaskade ein, an deren Ende der Übergang des Zellzyklus aus der G1-Phase in die S-Phase steht. Somit kann die weitere Zellteilung und ein weiteres dereguliertes Zellwachstum unterbunden werden, wenn dieses Schlüsselenzym gehemmt wird. KDR ist ein Tyrosin-Kinase-Rezeptor, der beim Wachstum von Endothel und bei der Angiogenese eine Schlüsselrolle spielt und somit ebenfalls bei der Tumorentstehung beteiligt. CDK4 und KDR stellen somit wichtige therapeutische Zielmoleküle für Krebserkrankungen und andere proliferative Erkrankungen dar. Im Test wird anhand der Phosphorylierung eines spezifischen Peptid-Substrates die Aktivität der CDK4-und KDR-Kinasen gemessen. Neben der inhibierenden Aktivität auf die genannten Kinasen, wurden auch weitere Kinasen die ebenfalls an der Krebsentstehung bzw. in der Entzündungskaskade beteiligt sind, durch die Klainetine gehemmt.

Aufgrund ihrer wertvollen pharmakologischen Eigenschaften eignen sich die erfindungsgemäßen Verbindungen zur speziellen Anwendung als Arzneimittel in der Human- und/oder Tiermedizin. Die erfindungsgemäßen Verbindungen können gegen Krebserkrankungen verwendet werden, insbesondere als Chemotherapeutika. Aufgrund ihrer cytostatischen Eigenschaften, insbesondere ihrer starken Antitumor-Aktivität sowie einer antimikrobiellen Wirkung können sie insbesondere als Cytostatika gegen maligne Entartungen bei Tieren sowie beim Menschen eingesetzt werden.

Bei Tumorzellen, die Resistenzen gegen herkömmliche Mittel ausgebildet haben, besitzen nur neue Agenzien eine therapeutisch ausreichende Wirkung. Die erfindungsgemäßen Klainetine und chemische Derivate davon der Formel I und II weisen somit potentiell eine hervorragende Wirkung auch gegen diese Problem-Zelltypen auf.

Die Erfindung betrifft auch pharmazeutische Zubereitungen, die eine oder mehrere der erfindungsgemäßen Klainetine und/oder chemische Derivate davon enthalten. Bevorzugt ist die Verwendung in Mischung mit geeigneten Hilfsstoffen oder Trägermaterial. Als Trägermaterial können beim Menschen alle pharmakologisch verträglichen Trägermaterialien und/oder Hilfsstoffe verwendet werden.

Die Erfindung betrifft auch ein Verfahren zur Herstellung eines erfindungsgemäßen Arzneimittels, das dadurch gekennzeichnet ist, dass man mindestens eine der erfindungsgemäßen Verbindungen mit einem pharmazeutisch geeigneten und physiologisch verträglichen Träger und gegebenenfalls weiteren geeigneten Wirk, Zusatz- oder Hilfsstoffen in eine geeignete Darreichungsform bringt.

Die erfindungsgemäßen Arzneimittel werden im allgemeinen oral, lokal oder parenteral verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z.B. Magnesiumcarbonat, Titandioxid, Laktose, Mannit und andere Zucker, Talkum, Micheiweiß, Gelatine, Stärke, Vitamine, Cellulose und ihre Derivate, tierische oder pflanzliche Öle, Polyethylenglykole und Lösungsmittel, wie etwa steriles Wasser, Alkohole, Glycerin und mehrwertige Alkohole genannt.

Gegebenenfalls können die Dosierungseinheiten für die orale Verabreichung mikroverkapselt werden, um die Abgabe zu verzögern oder über einen längeren Zeitraum auszudehnen, wie beispielsweise durch Überziehen oder Einbetten des Wirkstoffs in Teilchenform in geeignete Polymere, Wachse oder dergleichen.

Vorzugsweise werden die pharmazeutischen Präparate in Dosierungseinheiten hergestellt und verabreicht, wobei jede Einheit als aktiven Bestandteil eine bestimmte Dosis einer oder mehrerer Verbindungen der erfindungsgemäßen Klainetine und/oder chemische Derivate davon enthält. Bei festen Dosierungseinheiten wie Tabletten, Kapseln und Suppositorien kann diese Dosis bis zu etwa 500 mg, bevorzugt jedoch etwa 0,1 bis 200 mg, und bei Injektionslösungen in Ampullenform bis zu etwa 500 mg, vorzugsweise aber etwa 0,1 bis 100 mg, pro Tag betragen. Die zu verabreichende Tagesdosis ist abhängig vom Körpergewicht, Alter, Geschlecht und Zustand des Patienten. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber in mehreren kleineren Dosierungseinheiten als auch durch Mehrfachgabe unterteilter Dosen in bestimmten Intervallen erfolgen.

In den sich anschließenden Beispielen wird die Erfindung weiter erläutert. Prozentangaben beziehen sich auf das Gewicht. Mischungsverhältnisse bei Flüssigkeiten beziehen sich auf das Volumen, wenn keine anderen Angaben gemacht wurden.

### Beispiele

### Beispiel 1 Herstellung eines Primärextraktes von Uvaria klaineri

Blätter von Uvaria klaineri werden im frischen Zustand gesammelt und anschließend bei ca. 40°C luftgetrocknet. Nach dem Trocknen werden 100 g Trockenmaterial gemahlen und mit 1 I Methanol bei 40°C für 8h unter Rühren extrahiert. Nach Beenden der Extraktion werden die Pflanzenreste abfiltriert und der methanolische Extrakt bis nahezu zur Trockene unter Vakuum verdampft. Der Rückstand wird mit etwas Wasser nochmals resuspendiert und anschließend gefriergetrocknet. Der so entstandene Primärextrakt kann bei 4° oder -20° aufbewahrt werden oder zur weiteren Isolierung wie in Beispiel 3 verwendet werden. Zum Testen der biologischen Aktivität werden aus dem Primärextrakt mittels Chromatographie an Polyamid und an Polystyrol-Adsorberharz Tannine und andere stark hydrophile bzw. lipophile Störsubstanzen entfernt.

### Beispiel 2

Pflanzenproduktion: Sammeln der Samen, Säen, Wachstums- und Emte-Bedingungen.

Samen von Uvaria klaineri werden nach der Reifung gesammelt und können zur weiteren Anzucht der Pflanzen im Gewächshaus ausgesät werden. Die optimale Temperatur beträgt ca. 28° C bei einer Luftfeuchtigkeit von 70-90 %. Die Pflanzen werden mehrere Wochen bis Monate, vorzugsweise etwa 4-6 Monate kultiviert bis zur Ernte der Blätter.

### Beispiel 3

### Isolierung des Klainetin-Gemisches aus der Pflanze Uvaria klaineri.

Nach der Ernte von Uvaria klaineri werden 200 g getrocknete Blätter, gewonnen nach Beispiel 1, in einer Mühle zerkleinert, mit 2 Liter Methanol 16 Stunden gerührt und anschließend filtriert. Die wirkstoffhaltige, methanolische Lösung wird im Vakuum konzentriert; die Trockenmasse beträgt 3.05 g. Das Konzentrat wird auf eine vorbereitete, 412 mL ®MCI GEL, CHP20P-enthaltende Säule aufgetragen. Eluiert wird mit einem Gradienten von 10% Acetonitril in Wasser nach 90% Acetonitril in Wasser. Der Säulendurchfluß (50 mL pro Minute) wird fraktioniert aufgefangen (je 50 mL) und die Klainetine enthaltenden Fraktionen 31 bis 32 (Klainetin A) bzw. 51-56 (Klainetin B) werden zusammengefasst. Konzentrieren im Vakuum und Gefriertrocknung ergeben 26 mg (Klainetin A Rohprodukt) bzw. 70 mg (Klainetin B Rohprodukt) braunen Pulvers.

### Beispiel 4

### Reinigung des Klainetin A-Komponente auf Reverse Phase RP-18.

Es wird eine 19,7 mL fassende präparative HPLC-Säule (1 cm (ID) x 25 cm H) mit ®Superspher 100 RP-18 e (E. Merck, Darmstadt), gefüllt und die 26 mg des Klainetin A-Rohgemisches, gewonnen nach Beispiel 3, aufgetragen. Eluiert wird mit 30 % Acetonitril in 0,01 M wässriger Ammoniumacetat-Lösung, pH 7, nachdem die beladene Säule vorher mit 140 mL 25% Acetonitril in Wasser und dann mit 60 mL 30 % Acetonitril in Wasser gewaschen worden ist. Der Säulendurchfluss beträgt 10 ml / Minute, es werden Fraktionen von je 10 ml Inhalt gesammelt. In den Fraktion 25 und 26 befindet sich das Klainetin A. Nach dem Konzentrieren im Vakuum und Gefriertrocknung werden 9,8 mg Klainetin A in > 97% Reinheit ausgewogen: ESI + MS: 393.4 Da (M+H)⁺, 431.2 Da (M + K)⁺; ESI - MS: 391.5 Da (M - H)⁻

### Beispiel 5

### Charakterisierung des Klainetin A.

Aussehen: in polaren organischen Lösungsmitteln lösliche, jedoch in Wasser nur wenig lösliche, hellgelbe Substanz. Die Verbindung ist stabil im neutralen und mildsauren Milieu, jedoch unbeständig im alkalischen und stark-sauren Bereich.
UV-Maxima: 207, 286, 318 (Sh) nm in Wasser-Acetonitril (8:2), pH 2 sowie 253, 288 (Sh) und 325 nm in Wasser-Acetonitril (8:2) bei pH 7.
Durch hochauflösende Massenspektrometrie wurde für (M + H)⁺ folgendes Molekulargewicht gefunden: 393,13394 Da, entsprechen der Summenformel für das Klainetin A von C₂₃H₂₀O₆. Durch Elektronenspray - lonisation (ESI, positiv) werden mittels MS/MS-Fragmentierung folgende Ionen erhalten: 393, 289, 283, 271, 131 und 123 Da. Durch Elektronenspray - lonisation (ESI, negativ) werden mittels MS/MS-Fragmentierung folgende Ionen erhalten: 391, 269 und 109 Da
NMR-Signale: siehe Tabelle 1.

**Tab. 1: NMR-chemische Verschiebungen von Klainetin A, c = 2 mg /ml, 300 K in DMSO**

| Position | δ(¹³C) | m(¹³C) | δ(¹H) | ⁿJ_{CH} | ⁿJ_{HH} |
|---|---|---|---|---|---|
| 2 | 77.46 | d | 5.448 | 7.33,2.869 | 2.867, |
| | | | | | 2.771 |
| 3 | 44.59 | t | 2.867, | - | 5.448 |
| | | | 2.771 | | |
| 4 | 187.62 | s | - | 2.867, 2.771, 5.448 | - |
| 4a | 104.37 | s | - | 6.17 | - |
| 5 | 159.97 | s | - | 6.17, 3.747 | - |
| 5-OMe | 55.30 | q | 3.747 | - | - |
| 6 | 106.44 | s | - | 6.17, 3.747 | - |
| 7 | 161.60 | s | - | 3.747 | - |
| 7-OH | - | - | ? | - | - |
| 8 | 92.99 | d | 6.17 | - | - |
| 8a | 162.88 | sbr | - | - | - |
| 9 | 139.41 | s | - | 2.869, 5.448, 7.34, 7.30 | - |
| 10 2C | 125.85 | d | 7.33 | 7.33, 7.30, 5.448 | (7.34) |
| 11 2C | 128.32 | d | 7.34 | 7.34 | (7.30, 7.33) |
| 12 | 127.87 | d | 7.30 | 7.33 | 7.34 |
| 13 | 22.15 | t | 3.75 | 6.17 | - |
| 14 | 127.52 | s | - | 3.75, 6.43 | - |
| 15 | 142.93 | s | - | 6.174, 6.566, 3.757 | - |
| 16 | 144.61 | s | - | 6.428 | - |
| 17 | 112.58 | d | 6.566 | 6.174 | 6.428 |
| 18 | 118.16 | d | 6.428 | - | 6.566, |
| | | | | | 6.174 |
| 19 | 118.43 | d | 6.174 | 6.566,3.757 | 6.428 |

### Beispiel 6

### Reinigung der Klainetin B-Komponente auf Reverse Phase RP-18.

Es wird eine 19.7 mL fassende präparative HPLC-Säule (1 cm (ID) x 25 cm H) mit ®Superspher 100 RP-18 e (E. Merck, Darmstadt), gefüllt und die 70 mg des Klainetin B-Rohgemisches, gewonnen nach Beispiel 3, aufgetragen. Eluiert wird mit einem Gradienten von 35 % Acetonitril in 0,01 M wässriger Ammoniumacetat-Lösung, pH 7.3 nach 50% Acetonitril in wässriger Ammoniumacetat-Lösung, pH 7.3. Der Säulendurchfluss beträgt 10 ml / Minute, es werden Fraktionen von je 10 ml Inhalt gesammelt. In den Fraktion 10 bis 14 befindet sich das Klainetin B. Nach dem Konzentrieren im Vakuum und Gefriertrocknung werden 38 mg Klainetin B ausgewogen:
ESI + MS: 393.3 Da (M+H)⁺, ESI - MS: 391.5 Da (M - H)⁻

### Beispiel 7

### Charakterisierung des Klainetin B

Aussehen: in polaren organischen Lösungsmitteln lösliche, jedoch in Wasser nur wenig lösliche, hellgelbe Substanz. Die Verbindung ist stabil im neutralen und mildsauren Milieu, jedoch unbeständig im alkalischen Bereich.
UV-Maxima: 215 (Sh), 348 nm in Wasser-Acetonitril (8:2), pH 2 sowie 219 (Sh), 302 und 385 nm in Wasser-Acetonitril (8:2), pH 7.
Durch hochauflösende Massenspektrometrie wurde für (M + H)⁺ folgendes Molekulargewicht gefunden: 393,13391 Da, entsprechen der Summenformel für das Klainetin B von C₂₃H₂₀O₆. Durch Elektronenspray - lonisation (ESI, positiv) werden mittels MS/MS-Fragmentierung folgende lonen erhalten: 393, 289, 283, 271, 131 und 123 Da. Durch Elektronenspray - lonisation (ESI, negativ) werden mittels MS/MS-Fragmentierung folgende lonen erhalten: 391, 281, 269 und 109 Da.

Klainetin B hat die Summenformel C₂₃H₂₀O₆, das Molekulargewicht ist 392,41 Da. NMR-Signale: siehe Tabelle 2.

**Tab. 2: NMR-chemische Verschiebungen von Klainetin B, c = 4 mg /ml, 300 K in DMSO**

| Position | δ(¹³C) | m(¹³C) | δ(¹H) | ⁿJCH | ⁿJ_{HH} |
|---|---|---|---|---|---|
| 2 | 141.50 | d | 7.714 | 7.73 | 7.961 |
| 3 | 127.49 | D | 7.961 | 7.714 | 7.714 |
| 4 | 191.59 | s | - | 7.961, 7.714, 6.16 | - |
| 4a | 104.55 | s | - | 6.16, 14.47 | - |
| 5 | 165.05 | s | - | 14.47, 3.73 | - |
| 5-OH | - | - | 14.47 | - | - |
| 6 | 106.08 | s | - | 6.16, 3.73, 14.47 | - |
| 7 | 163.78 br | s | - | 6.16,3.73 | - |
| 7-OH | - | - | ? | - | - |
| 8 | 91.18 | d | 6.16 | (3.92) | - |
| 8a | 160.99 | S | - | 3.92,6.16 | - |
| 9 | 134.99 | s | - | 7.96, 7.714, 7.47 | - |
| 10 2C | 128.31 | d | 7.73 | 7.73, 7.46 | 7.47 |
| 11 2C | 129.01 | d | 7.47 | 7.47 | 7.73 |
| 12 | 130.22 | d | 7.46 | 7.73 | - |
| 15 | 21.60 | t | 3.73 | 6.15 | - |
| 16 | 127.50 | s | - | 6.43,3.73 | - |
| 17 | 142.84 | s | - | 3.73, 6.57, 6.43, (6.15), (9.09) | - |
| 17-OH | - | - | 9.09 s br | - | - |
| 18 | 144.60 | s | - | 6.57, (6.15), (3.73) | - |
| 19 | 112.65 | d | 6.57 | 6.15, (3.73) | 6.43 |
| 20 | 118.24 | d | 6.43 | - | 6.57, 6.15 |
| 21 | 118.37 | d | 6.15 | 6.57,3.73 | 6.43 |

### Beispiel 8

### Untersuchung der cytostatischen Wirksamkeit (IC₅₀ Bestimmung)

Zur Bestimmung der cytostatischen Aktivitäten wird mit gereinigten Enzymen in 384 well Mikrotiterplatten (Coated FlashPlates NEN Life Science) gearbeitet. Die Enzymaktivitäten werden mittels der Phosphorylierung eines jeweils spezifischen Peptidsubstrates bestimmt. Eine vorher vorbereitete Verdünnungsreihe der Klainetine mit Konzentrationen von 100, 50, 25, 12.5, 6.25, 3.125, 1.5625, 0.7813, 0.3906, 0.195, 0.094, 0.047 und 0 µM wird in der entsprechenden Reihenfolge in die Versuchsansätze pipettiert. Anschließend erfolgt die Zugabe des Reaktionsgemisches (radioaktiv markiertes ATP, Puffertösung pH 7,4 und Enzymlösungen) und Inkubation für 1 h bei RT.

### Beschreibung Reaktionsansätz CDK4:

Kinasepuffer: 50mM HEPES, 10mM MgCl₂, 2.5mM EGTA pH8.0, 10mM β-Glycerolphosphate, 1 mM Ortho-vanadate, 1 mM Sodium Fluoride, and 1mM DTT Biotinyliertes Peptidsubstrat zum Coaten der FlashPlates (Lösung 1 mg/ml in PBS Puffer)
ATP-Lösung: 100µCi/ml 33P-γ- ATP und 10µM ATP
CyclinD1/ CDK4 Enzymlösung: 100mg/ml in Kinasepuffer
Waschlösung: 3% Phosphorsäure
Endvolumen: 50 µl
30µl verdünnte Klainetin-Lösung, 20 µl ATP/Enzymlösung (Endkonz. 1µCi 33P-γ-ATP, 2 µM ATP und 1 µg Enzym
2h Inkubation bei RT, anschließend 3x Waschen mit 80µl Waschlösung und Messung in MicroBeta Counter (Wallac), 30sec

### Beschreibung Reaktionsansatz KDR

Kinasepuffer: 50mM MOPS, pH7.4, 10mM MgCl₂,2mM DTT, 2.5mM EGTA, 10mM β-Glycerolphosphat, 1 mM Ortho-Vanadate, and 1 mM Sodium Fluoride.
Peptidsubstrat: PLCγ1
ATP-Lösung: 25µCi/ml 33P-γ- ATP und 12,5 µM ATP
KDR-Enzymlösung: 3,5 µg/ml in Kinasepuffer
Waschlösung: PBS (ohne Mg²⁺, Ca²⁺)
Endvolumen 50 µl
10µl verdünnte Klainetin-Lösung
20 µl Enzymlösung (3,5 µg/ml; 70 ng/well)
20 µl ATP-lösung (Endkonzentration0,5µCi und 5µM ATP/well)
Inkubation bei RT für 1 h, anschließend 3x Waschen mit je 75µl Waschlösung und Messung in MicroBeta Counter (Wallac) für 30s.
Die Aktivität der Kinasen (CDK4 oder KDR) wird über den Einbau von radioaktivem Phosphat in das Substrat aus ATP gemessen und daraus die inhibierende Wirkung der Klainetine (IC₅₀) berechnet.
IC₅₀ Werte der Klainetine A und B

| **Verbindung** | **IC**_{**50**} **CDK4** (µg/l) | **IC**_{**50**}**CDK4** (µM/l) | **IC**_{**50**} **CDK2** (µg/ml) | **IC**_{**50**} **CDK2** (µM/l) | **IC**_{**50**} **KDR** (µg/l) | **IC**_{**50**} **KDR** (µM/l) |
|---|---|---|---|---|---|---|
| Klainetin A | 0,82 | 2,09 | 8,45 | 21,53 | 2,73 | 6,96 |
| Klainetin B | 0,45 | 1,14 | 2,37 | 6,04 | 2,54 | 6,48 |

## Patentansprüche

1. Verbindung der allgemeinen Formel I 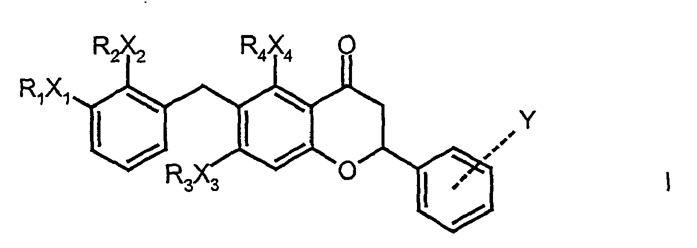 und der Formel II 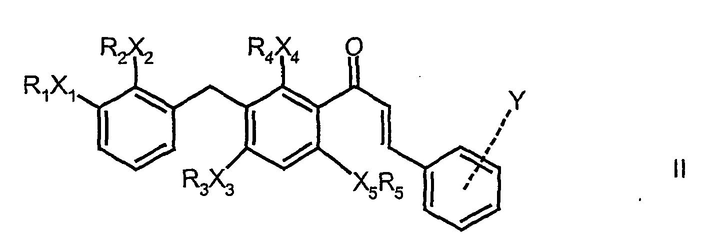 worin
R₁, R₂, R₃, R₄ und R₅ unabhängig von einander, jeweils H, C₁-C₆-alkyl, C₂-C₆-alkenyl oder C₂-C₆-Alkinyl, gegebenenfalls mit einem oder mehreren OH substituiert;
X₁, X₂,X₃, X₄ und X₅ unabhängig voneinander O, NH, N-C₁-C₆-Alkyl, N-C₂-C₆-Alkenyl, N-C₂-C₆-Alkinyl, Acyl, Aryl oder S;
Y eine oder mehrere H, Halogen, OH, O-C₁-C₆-Alkyl, O-C₂-C₆-Alkenyl, O-C₂-C₆-Alkinyl, NH₂, NH-C₁-C₆-Alkyl, NH-C₂-C₆-Alkenyl, NH-C₂-C₆-A)kinyl, NH-acyl, SH, S-C₁-C₆-Alkyl, S-C₂-C₆-Alkenyl, S-C₂-C₆-Alkinyl, Acyl oder Aryl; bedeuten,
in allen ihren stereochemischen Formen und Gemische dieser Formen in jedem Verhältnis, sowie deren physiologisch verträgliche Salze.

2. Verbindung der Formel I oder II gemäß Anspruch 1 worin X₁, X₂, X₃ und X₄, unabhängig voneinander, O, NH oder S bedeuten, und R₁, R₂, R₃, R₄ und R₅, unabhängig voneinander, H oder C₁-C₆-Alkyl bedeuten, sowie deren physiologisch verträgliche Salze.

3. Verbindung der Formel IA 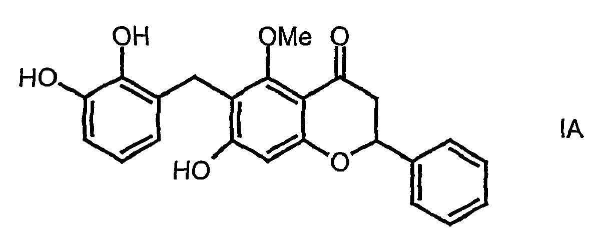 in allen ihren stereochemischen Formen und Gemische dieser Formen in jedem Verhältnis, sowie deren physiologisch verträglichen Sälzen.

4. Verbindung der Formel IIA 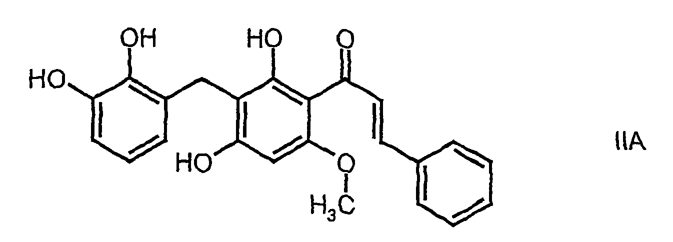 in allen ihren stereochemischen Formen und Gemische dieser Formen in jedem Verhältnis, sowie deren physiologisch verträglichen Salzen.

5. Verbindung der Formel I gemäß einem oder mehreren der Ansprüche 1 - 4, erhältlich durch Extraktion der Pflanze Uvaria klaineri oder einer ihrer Varianten oder Mutanten, oder Züchtung der Pflanze Uvaria klaineri bis sich eine Verbindung der Formel IA und/oder IIA in der Pflanze anhäuft und durch anschließende Isolierung der Verbindung und gegebenenfalls Umwandlung in chemische Derivate, sowie deren physiologisch verträglichen Salze.

6. Verfahren zur Herstellung einer Verbindung der Formel I und II oder ein physiologisch verträgliches Salz davon gemäß einem oder mehreren der Ansprüche 1 - 4, **dadurch gekennzeichnet, dass** die Pflanze Uvaria klaineri oder einer ihrer Varianten oder Mutanten unter geeigneten Bedingungen in einem Kulturmedium kultiviert wird, bis sich eine Verbindung der Formel IA und/oder IIA in der Pflanze anhäuft und anschließend aus dem Pflanzenmaterial isoliert und gegebenenfalls in chemische Derivate und physiologisch verträglichen Salze umgewandelt wird.

7. Verfahren gemäß Anspruch 6, **dadurch gekennzeichnet, dass** die Pflanzenkultur unter tropischen oder subtropischen Bedingungen durchgeführt wird.

8. Verbindung der Formel I oder ein physiologisch verträgliches Salz davon gemäß einem oder mehreren der Ansprüche 1-5 zur Verwendung als Arzneimittel.

9. Verbindung der Formel I oder ein physiologisch verträgliches Salz davon gemäß einem oder mehreren der Ansprüche 1-5 zur Verwendung als Arzneimittel zur Hemmung von Cyclin-abhängigen Kinasen (CDK).

10. Verbindung der Formel I oder ein physiologisch verträgliches Salz davon gemäß einem oder mehreren der Ansprüche 1-5 zur Verwendung als Cytostatikum.

11. Pharmazeutische Zubereitung mit einem Gehalt an mindestens einer Verbindung der Formel t oder eines physiologisch verträglichen Salzes davon gemäß einem oder mehreren der Ansprüche 1-5.

12. Verfahren zur Herstellung von pharmazeutischen Zubereitungen gemäß Anspruch 11, **dadurch gekennzeichnet, dass** man mindestens eine Verbindung der Formel I oder ein physiologisch verträgliches Salz davon gemäß einem oder mehreren der Ansprüche 1-5 mit geeigneten Hilfs- und/oder Trägerstoffen in eine geeignete Darreichungsform bringt.

13. Verwendung von Uvaria klaineri zur Gewinnung von Cytostatika der Formeln I oder II gemäss Anspruch 1.

## Claims

1. A compound of the formula I 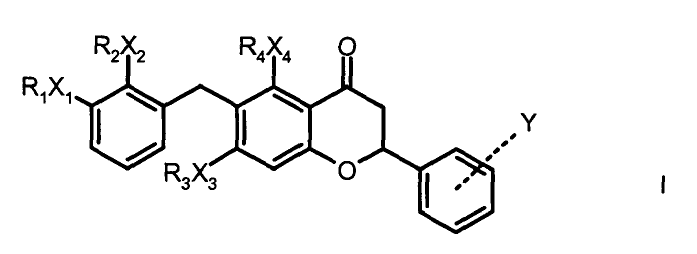 and the formula II 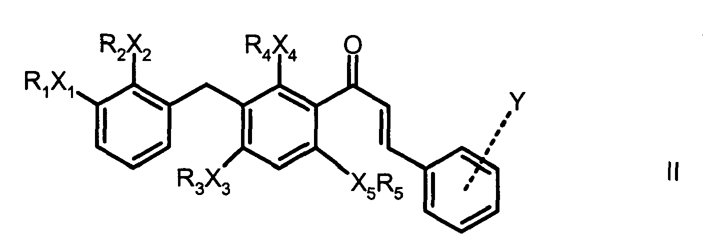 in which
R₁, R₂, R₃, R₄ and R₅ independently of one another are in each case H, C₁-C₆-alkyl, C₂-C₆-alkenyl or C₂-C₆-alkynyl, unsubstituted or substituted with one or more OH;
X₁, X₂ ,X₃, X₄ and X₅ independently of one another are O, NH, N-C₁-C₆-alkyl, N-C₂-C₆₋alkenyl, N-C₂-C₆-alkynyl, acyl, aryl or S;
Y is one or more H, halogen, OH, O-C₁-C₆-alkyl, O-C₂-C₆-alkenyl, O-C₂-C₆-alkynyl, NH₂, NH-C₁-C₆-alkyl, NH-C₂-C₆-alkenyl, NH-C₂-C₆-alkynyl, NH-acyl, SH, S-C₁-C₆-alkyl, S-C₂-C₆-alkenyl, S-C₂-C₆-alkynyl, acyl or aryl;
in all its stereochemical forms, and mixtures of said forms in any ratio, and also physiologically tolerated salts thereof.

2. A compound of the formula I or II as claimed in claim 1, in which X₁, X₂, X₃ and X₄ independently of one another are O, NH or S, and R₁, R₂, R₃, R₄ and R₅ independently of one another are H or C₁-C₆-alkyl, and also physiologically tolerated salts thereof.

3. A compound of the formula IA 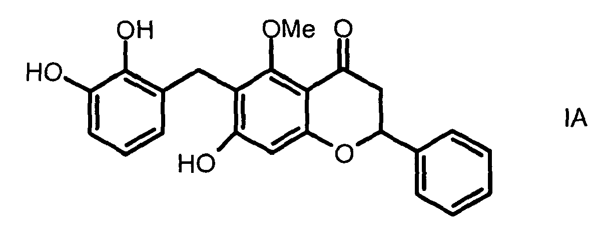 in all its stereochemical forms, and mixtures of said forms in any ratio, and also physiologically tolerated salts thereof.

4. A compound of the formula IIA 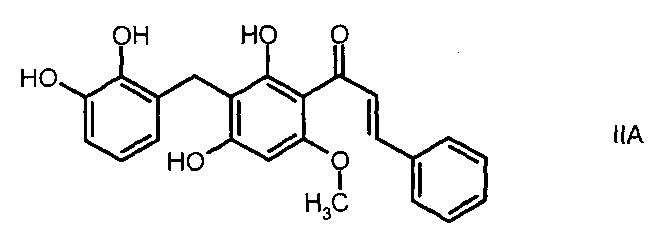 in all its stereochemical forms, and mixtures of said forms in any ratio, and also physiologically tolerated salts thereof.

5. A compound of the formula I as claimed in one or more of claims 1-4, obtainable by extracting the plant Uvaria klaineri or any of its variants or mutants, or growing the plant Uvaria klaineri, until a compound of the formulae IA and/or IIA accumulates in the plant, and by subsequent isolation of the compound and, where appropriate, conversion into chemical derivatives, and physiologically tolerated salts thereof.

6. A method for preparing a compound of the formulae I and II or a physiologically tolerated salt thereof as claimed in one or more of claims 1 - 4, which comprises culturing the plant Uvaria klaineri or any of its variants or mutants under suitable conditions in a culture medium, until a compound of the formulae IA and/or IIA accumulates in the plant, is subsequently isolated from the plant material and, where appropriate, is converted into chemical derivatives and physiologically tolerated salts.

7. The method as claimed in claim 6, wherein the plant is cultured under tropical or subtropical conditions.

8. A compound of the formula I or a physiologically tolerated salt thereof as claimed in one or more of claims 1 - 5 for use as pharmaceutical.

9. A compound of the formula I or a physiologically tolerated salt thereof as claimed in one or more of claims 1 - 5 for use as pharmaceutical for inhibition of cyclin-dependent kinases (CDK).

10. A compound of the formula I or a physiologically tolerated salt thereof as claimed in one or more of claims 1 - 5 for use as cytostatic.

11. A pharmaceutical preparation comprising at least one compound of the formula I or a physiologically tolerated salt thereof as claimed in one or more of claims 1-5.

12. A method for producing pharmaceutical preparations as claimed in claim 11, which comprises bringing at least one compound of the formula I or a physiologically tolerated salt thereof as claimed in one or more of claims 1 - 5 into a suitable administration form using suitable excipients and/or carriers.

13. The use of Uvaria klaineri for producing cytostatics of the formula I or II as claimed in claim 1.

## Revendications

1. Composé de formule générale I 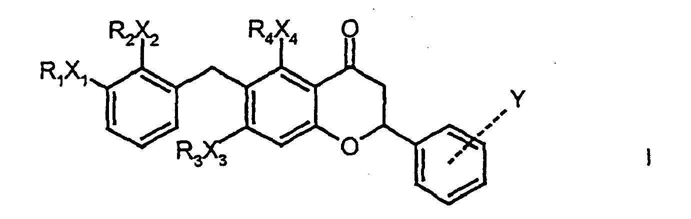 et de formule II 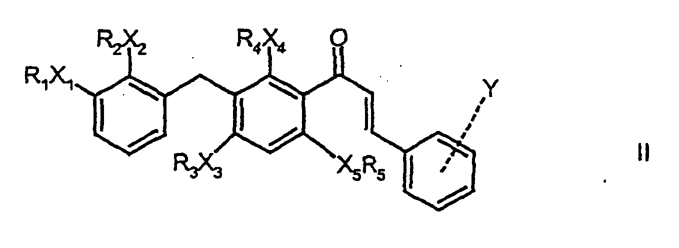 dans lesquelles
R₁, R₂, R₃, R₄ et R₅ représentent chacun, indépendamment les uns des autres, H, un groupe alkyle en C₁-C₆, alcényle en C₂-C₆ ou alcynyle en C₂-C₆, éventuellement substitué par un ou plusieurs groupes OH ;
X₁, X₂, X₃, X₄ et X₅ représentent, indépendamment les uns des autres, O, NH, un groupe alkyle en C₁-C₆, N-alcényle en C₂-C₆, N-alcynyle en C₂-C₆, acyle, aryle ou S ;
Y représente un ou plusieurs atomes d'hydrogène ou d'halogène, ou groupes OH, O-alkyle en C₁-C₆, O-alcényle en C₂-C₆, O-alcynyle en C₂-C₆, NH₂, NH-alkyle en C₁-C₆, NH-alcényle en C₂-C₆, NH-alcycnyle en C₂-C₆, NH-acyle, SH, S-alkyle en C₁-C₆, S-alcényle en C₂-C₆, S-alcynyle en C₂-C₆, acyle ou aryle,
sous toutes ses formes stéréochimiques et en tous mélanges de ces formes en tout rapport, et sels physiologiquement acceptables d'un tel composé.

2. Composé de formule I ou II selon la revendication 1, dans lequel X₁, X₂, X₃ et X₄ représentent, indépendamment les uns des autres, O, NE ou S, et R₁, R₂, R₃, R₄ et R₅ représentent, indépendamment les uns des autres, H ou un groupe alkyle en C₁-C₆, et sels physiologiquement acceptables d'un tel composé.

3. Composé de formule IA 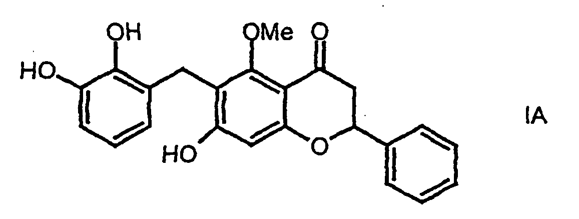 sous toutes ses formes stéréochimiques et en tous mélanges de ces formes en tout rapport, et sels physiologiquement acceptables d'un tel composé.

4. Composé de formule IIA 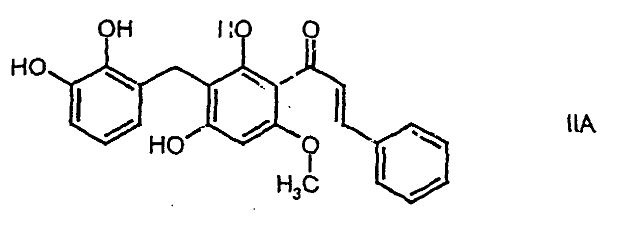 sous toutes ses formes stéréochimiques et en tous mélanges de ces formes en tout rapport, et sels physiologiquement acceptables d'un tel composé.

5. Composé de formule I selon une ou plusieurs des revendications 1 à 4, pouvant être obtenu par extraction de la plante *Uvaria klaineri* ou d'un de ses variants ou mutants, ou culture de la plante *Uvaria klaineri* jusqu'à ce que s'accumule dans la plante un composé de formule IA et/ou IIA et par isolement subséquent du composé et éventuellement transformation en dérivés chimiques, ainsi que de sels physiologiquement acceptables d'un tel composé.

6. Procédé pour la préparation d'un composé de formule I ou II ou d'un sel physiologiquement acceptable d'un tel composé, selon une ou plusieurs des revendications 1 à 4, **caractérisé en ce qu'**on cultive dans des conditions appropriées la plante *Uvaria klaineri* ou un de ses variants ou mutants, jusqu'à ce que s'accumule dans la plante un composé de formule IA et/ou IIA et ensuite on l'isole à partir de la matière végétale et éventuellement on le transforme en dérivés chimiques et sels physiologiquement acceptables.

7. Procédé selon la revendication 6, **caractérisé en ce que** la culture de la plante est effectuée dans des conditions tropicales ou subtropicales.

8. Composé de formule I ou sel physiologiquement acceptable d'un tel composé, selon une ou plusieurs des revendications 1 à 5, pour utilisation en tant que médicament.

9. Composé de formule I ou sel physiologiquement acceptable d'un tel composé, selon une ou plusieurs des revendications 1 à 5, pour utilisation en tant que médicament destiné à l'inhibition de kinases dépendant de la cycline (CDK) .

10. Composé de formule I ou sel physiologiquement acceptable d'un tel composé, selon une ou plusieurs des revendications 1 à 5, pour utilisation en tant qu'agent cytostatique.

11. Composition pharmaceutique ayant une teneur en au moins un composé de formule ou un sel physiologiquement acceptable d'un tel composé, selon une ou plusieurs des revendications 1 à 5.

12. Procédé pour la production de préparations pharmaceutiques selon la revendication 11, **caractérisé en ce qu'**on met sous une forme d'administration appropriée au moins un composé de formule I ou un sel physiologiquement acceptable d'un tel composé, selon une ou plusieurs des revendications 1 à 5, avec des adjuvants et/ou véhicules appropriés.

13. Utilisation *d'Uvaria klaineri* pour l'obtention d'agents cytostatiques de formule I ou II selon la revendication 1.
